# EUROPEAN PATENT APPLICATION

(11) **EP 3 734 277 A1**
(43) Date of publication of application: **04.11.2020**
(21) Application number: 18893563.9
(22) Date of filing: 26.12.2018
(51) Int. Cl.: G01N 33/53, A61K 39/395, A61P 35/00

(54) **METHOD FOR ASSESSING MEDICINE**

(30) Priority: 27.12.2017 JP 2017250937
(71) Applicant: KONICA MINOLTA, INC., Tokyo 100-7015 (JP)
(72) Inventor: SETOGAWA Takeshi, Tokyo 100-7015 (JP); TAKAHASHI Masaru, Tokyo 100-7015 (JP)
(74) Representative: MFG Patentanwälte Meyer-Wildhagen Meggle-Freund Gerhard PartG mbB
(86) International application number: PCT/JP2018/047759
(87) International publication number: WO 2019/131727

(57) **Abstract**

An object of the present invention is to provide a method for accurately assessing effectiveness of a medicine that specifically recognizes a target molecule. The present invention can assess therapeutic effects of a medicine by an assessment method including step (P) of obtaining information on the target molecule, and further including at least one step selected from step (A) for obtaining information on a target-related molecule, step (B) for measuring blood concentration of the medicine in a subject, and step (C) of measuring a proportion of cells reached by the medicine (excluding, however, step (A) alone, and step (B) alone).

## Description

### Technical Field

The present invention relates to a method for assessing a medicine.

### Background Art

Although low-molecular-weight drugs have been widely used as therapeutic agents for cancer, a number of antibody medicines with higher stability in plasma, higher selectivity, higher therapeutic effect, and fewer side effects have been developed in recent years, and clinically used. Above all, a number of IgG-type antibody medicines are on the market, and these antibody medicines have an action mechanism of exerting anticancer activity due to antibody-dependent cellular cytotoxicity (ADCC), complement-dependent cytotoxicity (CDC), changes in signal transduction and the like when the IgG binds to an antigen expressed on cancer cells. Rituxan against CD20 antigen, cetuximab against EGFR antigen, Herceptin against HER2 antigen and the like have been approved as such antibody medicines for cancer treatment.

Further, aptamer medicines that bind to specific target molecules and have similar properties to antibodies have also attracted attention, and medicines that specifically recognize a target molecule are considered to be effective means for cancer treatment.

As a method for determining effectiveness of a medicine that specifically recognizes a target molecule, it is desired to specify information such as an interaction between the target molecule on target cancer cells and the medicine. For example, as a method for determining effectiveness of trastuzumab (Herceptin (registered trademark)) that is a typical antibody medicine in breast cancer treatment, analysis of expression information of HER2 protein that is a target molecule (antigen) of trastuzumab has been widely performed. As the method, for example, an immunohistochemistry (IHC) method for staining a protein of a target biological substance, a FISH (Fluorescence in situ hybridization) method for staining a target biological substance gene and the like are used. When the IHC method is used for breast cancer testing, a method of staining and detecting HER2 protein contained in a specimen using DAB (3,3'-diaminobenzidine) is widely used. However, since the criterion is a rough criterion based on only four scale with a staining level of scores 0 to 3, quantification is lacking, and further the criterion depends on the skill of the pathologist, which are clinically problematic. When the FISH method is used, a method of fluorescently staining a gene encoding HER2 protein on chromosome 17 using a probe for detecting the gene is widely used. Although the FISH method is a quantitative test method, it is not a method for directly assessing the amount or intracellular localization of HER2 protein.

In recent years, in order to more accurately assess the expression level and intracellular localization of proteins, a staining method for labeling a protein of interest with nano-sized fluorescent particles, for example, particles (Phosphor Integrated Dot: PID) obtained by integrating phosphors such as fluorochromes and quantum dots using a resin or the like as a matrix, has been proposed and put into practical use. The protein of interest is labeled using a phosphor integrated dot (sometimes referred to as "phosphor-integrated nanoparticle", "phosphor-containing particle" or the like in other documents), and an excitation light adapted to the phosphor integrated in the dot is irradiated, whereby the protein of interest can be observed as a bright spot having high luminance, and observation and imaging can be performed for a relatively long time because it is not easily discolored. Patent Literature 1 and Patent Literature 2 describe a method for performing immunostaining using a phosphor integrated dot.

Furthermore, Patent Literature 3 describes a tissue staining method performed by using an antibody (trastuzumab or the like) that is an antibody medicine labeled with a phosphor integrated dot and binding it to its target antigen (HER2 or the like), and further describes that such a method can be applied to a method for determining effectiveness of an antibody medicine.

### Citation List

### Patent Literature

Patent Literature 1: WO 2013/035703 A
Patent Literature 2: WO 2012/029752 A
Patent Literature 3: WO 2012/133047 A

### Summary of Invention

### Technical Problem

The present invention has been made in view of the above, and an object of the present invention is to provide a method for accurately assessing effectiveness of a medicine (hereinafter, also referred to as medicine X) that specifically recognizes a target molecule.

### Solution to Problem

The present inventor has found that it is possible to assess therapeutic effects of medicine X by performing an analysis by combining information on a target molecule, information on a target-related molecule, blood concentration of the medicine X, a proportion of cells reached by the medicine X, and the like.

That is, the present invention provides the method for assessing a medicine as follows.

### [Section 1]

A method for assessing a medicine that specifically recognizes a target molecule, including step (P) of obtaining information on the target molecule, and further including:
at least one step selected from the following steps (A), (B) and (C) (excluding, however, step (A) alone, and step (B) alone).
step (A) of obtaining information on a target-related molecule,
step (B) of measuring blood concentration of the medicine that specifically recognizes a target molecule in a subject, and
step (C) of calculating a proportion of cells reached by the medicine that specifically recognizes a target molecule.

### [Section 2]

The method for assessing a medicine according to section 1, wherein the information on a target-related molecule includes at least one of information on an expression level of the target-related molecule and information on localization of the target-related molecule.

### [Section 3]

The method for assessing a medicine according to section 1 or 2, wherein the information on a target molecule includes at least one of information on an expression level of the target molecule and information on localization of the target molecule.

### [Section 4]

The method for assessing a medicine according to any one of sections 1 to 3, wherein the target molecule is HER2, CTLA4, EGFR, PD-1, PDGFRA, PD-L1, VEGF, or VEGFR.

### [Section 5]

The method for assessing a medicine according to any one of sections 1 to 4, wherein the medicine that specifically recognizes a target molecule is an antibody medicine.

### [Section 6]

The method for assessing a medicine according to any one of sections 1 to 5, wherein the step (C) is a step of quantifying blood concentration of the antibody medicine using ELISA.

### [Section 7]

The method for assessing a medicine according to any one of sections 1 to 4, wherein the medicine that specifically recognizes a target molecule is a nucleic acid medicine.

### [Section 8]

The method for assessing a medicine according to section 7, wherein the target molecule is PDGF-B, C5, MCP-1, SDF-1, Hepcidin, RTP801, Nucleocapsid, transthyretin, or HSP47.

### Advantageous Effects of Invention

According to the assessment method of the present invention, it is possible to predict and assess therapeutic effects of the medicine X and obtain useful information for diagnosis or treatment using the medicine X, and a patient adapted for administration can be more accurately selected, and also an appropriate dosing regimen can be established.

### Brief Description of Drawings

Fig. 1 is a graph in which measurement results of blood concentration of trastuzumab performed in Experimental Example 1 are plotted. The results of high concentration group are each indicated by black circles, and the results of low concentration group are each indicated by white circles.

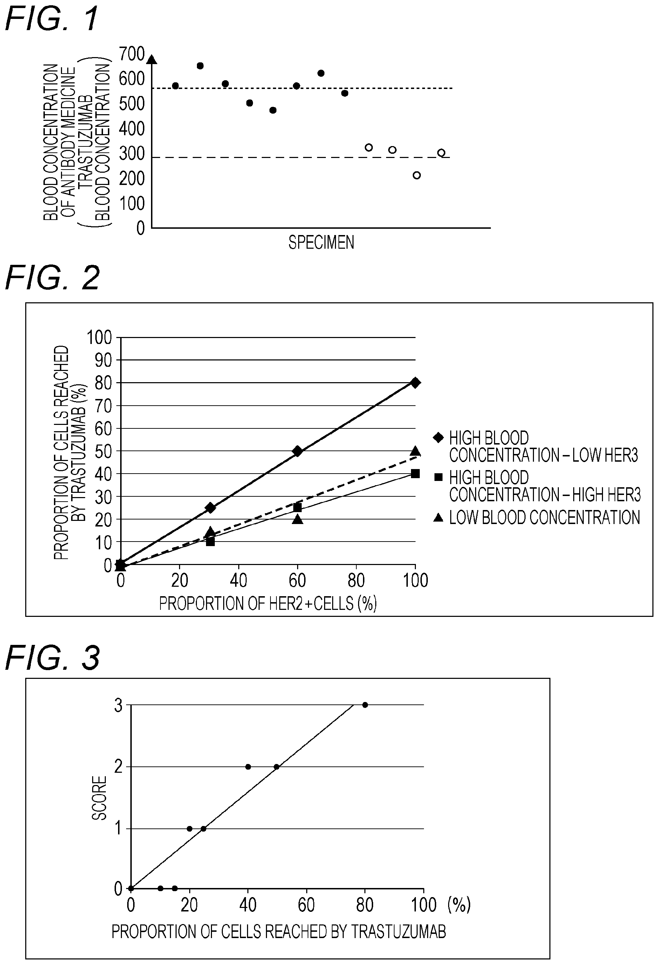

Fig. 2 is a graph obtained by dividing specimens into a high-concentration group and a low-concentration group based on trastuzumab blood concentration, further dividing the high-concentration group into a HER3 high expression group and a HER3 low expression group, and plotting a ratio of HER2 + cells and a proportion of cells reached by trastuzumab in each group. A solid line (thick) shows a linear approximation curve in the high blood concentration-HER3 low expression group, a solid line (thin) shows a linear approximation curve in the high blood concentration-HER3 high expression group, and a dotted line shows a linear approximation curve in the low blood concentration group.
Fig. 3 is a graph obtained by plotting the proportion of cells reached by trastuzumab and a treatment score of trastuzumab in each specimen, and linearly approximating the plot.

### Description of Embodiments

### <Medicine that specifically recognizes target molecule>

The medicine (medicine X) that specifically recognizes a target molecule in the present invention is a medicine utilizing a mechanism for recognizing a specific molecule such as a protein as a target, and typical examples thereof include antibody medicines, nucleic acid medicines, peptide aptamers, and the like. Among them, nucleic acid medicines or antibody medicines are preferable, and antibody medicines are more preferable, because they have high stability in blood of a subject.

The antibody medicine in the present invention is a drug containing an antibody (immunoglobulin) that recognizes an antigen as a main component. The term "antibody" herein includes not only full-length antibodies, but also antibody fragments such as Fab, F(ab)'2, Fv and scFv, and derivatives such as chimeric antibodies (humanized antibodies and the like), multifunctional antibodies, and Antibody-Drug Conjugate (ADC).

The nucleic acid medicine in the present invention is a medicine having a basic skeleton of a naturally-occurring or chemically-modified nucleotide, which acts directly on a living body without involving gene expression and is produced by chemical synthesis. Examples of the nucleic acid medicine adaptable to the present invention include nucleic acid aptamers, decoys, antisenses, siRNAs, ribozymes, miRNA-mimics, and the like, but nucleic acid aptamers are preferred because they can specifically recognize protein that is a target molecule.

Aptamers can be broadly classified into nucleic acid (DNA or RNA) aptamers and peptide aptamers. In the present invention, either of nucleic acid aptamers or peptide aptamers can be used, but nucleic acid aptamers are preferred because oligonucleic acids are easily available for aptamer medicine development.

### <Method for assessing medicine X>

The present invention relates to a method for assessing medicine X, including step (P) of obtaining information on a target molecule, and further including at least one step selected from step (A) for obtaining information on a target-related molecule, step (B) for measuring blood concentration of the medicine X in a subject, and step (C) of measuring a proportion of cells reached by the medicine X. However, the present invention does not include embodiments in which the step (A) alone or the step (B) alone is performed.

The steps (P) and (A) are steps performed on a specimen derived from a subject before administration of medicine X, and are each preferably performed on the same specimen (a tissue section or the like). The steps (B) and (C) are steps performed on a specimen derived from a subject after administration of medicine X. The specimen in each step is a specimen derived from the same subject. In other words, after administering the medicine X to the subject before administration of medicine X from which a specimen for performing the steps (P) and (A) was collected, a specimen for performing the steps (B) and (C) is collected.

The step (P), the step (A) and the step (C) in the present invention are each preferably performed by a staining method using a phosphor integrated dot (PID), from a viewpoint that the target molecule, target-related molecule, and medicine X can be observed in a "quantitative" manner but not in a "qualitative" manner, and particularly when the medicine X is an antibody medicine, these steps are preferably performed by an immunostaining method. Specifically, immunostaining may be performed using an IgG that specifically recognizes and binds to a target molecule, a target-related molecule and an antibody medicine, to which a PID is bound, or immunostaining may be performed using an IgG that specifically recognizes and binds to a target molecule, a target-related molecule and an antibody medicine and an IgG that specifically recognizes and binds to an antibody, to which a PID is bound.

When performing immunostaining using a PID in a plurality of steps of the step (P), the step (A) and the step (C), it is preferable to use a PID that emits fluorescence of a different wavelength in each step.

The subject in the present invention may be a human (a cancer patient or a person suspected of having cancer) or a non-human animal. An example of the non-human animal includes an experimental animal, typically, a tumor-bearing animal, and preferably, a tumor-bearing mouse. Specific examples of the tumor-bearing mouse include a PDX [Patient derived xenograft] model mouse, an Immuno-avatar model mouse, a Hemato-lymphoid humanized model mouse and an Immune-PDX model mouse, prepared by implanting cancer cells or tumor tissues collected from human into an acquired immunodeficient mouse, and a CDX [Cell-line derived xenograft] model mouse, prepared by implanting cultured cells derived from tumor cells collected from the subject into an acquired immunodeficient mouse, and the like.

The tumor-bearing mouse includes a zero-generation experimental animal implanted with a tumor (cancer) tissue or tumor cells collected from a human (cancer patient) or cultured cells derived from tumor cells removed from the patient, and a n-generation (n ≥ 1) experimental animal implanted with a tumor tissue grown in a body of a n-1 generation experimental animal or proliferated tumor cells, originated from the tumor tissue or tumor cells implanted in the zero-generation.

In addition, although the medicine X to be assessed in the present invention is not particularly limited, one containing cancer as an adaptive disease is preferable, and the medicine X may be medicine X already marketed and used clinically, or may be medicine X as a candidate medicine used in a clinical test or clinical trial.

Specific examples of commercially available antibody medicine include humanized anti-HER2 antibody trastuzumab (Herceptin (registered trademark)), anti-CD30 monoclonal antibody brentuximab, anti-VEGF monoclonal antibody bevacizumab, humanized anti-CD33 antibody gemtuzumab, humanized anti-PD-1 antibodies pembrolizumab and nivolumab, anti-CTLA-4 ipilimumab monoclonal antibodies, and the like.

Specific examples of nucleic acid aptamer medicine include E10030 (Fovista (registered trademark)) targeting PDGF-B, ARC1905 (Zimura (registered trademark)) targeting C5, NOX-E36 (Emaoticap Pegol) targeting MCP-1, NOX-A12 (Olaptesed Pegol) targeting SDF-1, NOX-H94 (Lezapteid Pegol) targeting Hepcidin, and the like.

Specific examples of siRNA medicine include PF-655 targeting RTP801, ALN-RSV01 targeting Nucleocapsid, ND-L02-s0201 targeting HSP47, and the like.

### <Step (P)>

In the present invention, the step (P) is a step of obtaining information on a target molecule in a specimen derived from a tumor tissue, and preferably contains at least one of information on an expression level of the target molecule and information on localization of the target molecule and more preferably contains both information. Examples of the information on an expression level of the target molecule include, in the specimen (tissue slide), the type, number and morphology of cells expressing the target molecule, an expression level per unit area or per cell of the target molecule in the specimen, a histogram and curve represented by the expression level of the target molecule per cell and the number of cells corresponding thereto, and the like. Examples of the information on localization of the target molecule include localization of the target molecule in the specimen, localization in a region of interest (ROI) of a specific cell group (for example, a cancer cell group), and the like. These information is preferably information based on information obtained as an image (including information converted as a digital image), and is further preferably quantitative information. The information on the expression state is not limited to any one of the above information, but may be a combination thereof.

The target molecule herein is a biomolecule that is a target of the medicine X, and is typically a protein (antigen) that specifically binds to an antibody, a nucleic acid or the like that is a component of the medicine X. The target molecule is not particularly limited as long as it is a protein that can be present in a tumor tissue, and examples thereof include an immune system protein, a cancer cell growth factor, a metastasis regulator, a vascular growth factor, a cytokine, a cancer cell growth control factor receptor, a metastasis regulator receptor, a vascular growth factor receptor, and a cytokine receptor, and the target molecule is preferably a molecule involved in a signaling pathway, and more preferably a molecule involved in a proliferation signaling pathway. In the present invention, specific examples of the target molecule include HER2, CTLA4, EGFR, PD-1, PDGFRA, PD-L1, VEGF, VEGFR, and the like. Moreover, when the medicine X is a nucleic acid medicine, specific examples of the target molecule include PDGF-B, C5, MCP-1, SDF-1, Hepcidin, RTP801, Nucleocapsid, transthyretin, HSP47, and the like.

### <Step (A)>

The step (A) in the present invention is a step of obtaining information on a target-related molecule using a specimen derived from a tumor tissue, and preferably contains at least one of information on an expression level of the target-related molecule and information on localization of the target-related molecule and more preferably contains both information. The step (A) may use the same specimen as the specimen used in the step (P), or may use another specimen, but it is preferable to perform the step (A) using the specimen used in the step (P).

In the method for assessing medicine X of the present invention, the step (A) is performed in combination with the step (P) and at least one, preferably both, of the steps (B) and (C) described below.

Examples of the information on an expression level of the target-related molecule, in the specimen (tissue slide), the type, number and morphology of cells expressing the target-related molecule, an expression level per unit area or per cell of the target-related molecule in the specimen, a histogram and curve represented by the expression level of the target-related molecule per cell and the number of cells corresponding thereto, and the like, and examples of the information on localization of the target-related molecule include localization of the target-related molecule in the specimen, localization in a region of interest (ROI) of a specific cell group (for example, a cancer cell group), and the like. These information is preferably information based on information obtained as an image (including information converted as a digital image), and is further preferably quantitative information. The information on the expression state is not limited to any one of the above information, but may be a combination thereof.

The target-related molecule herein is a molecule involved in a signaling pathway in which the target molecule is typically involved in a tumor cell, and is specifically selected as follows. When selecting HER2 as the target molecule, the target-related molecule is preferably selected from HER3, EGF, GRB2, HRAS, KRAS, CTNNB1, PIK3CA, PTPN11, EGFR, GRB7, and BTC. When selecting CTLA4 as the target molecule, the target-related molecule is preferably selected from CD86, CD80, FOXP3, FYN, PTPN11, LCK, NFATC2, AKT1, ICOSLG, and LYN. When selecting EGFR as the target molecule, the target-related molecule is preferably selected from EGF, KRAS, HRAS, GRB2, SHC1, TP53, CBL, PIK3CA, TGFA, and PTPN11. When selecting PD-1 as the target molecule, the target-related molecule is preferably selected from CD274, PDCD1LG2, HLA-DRB5, HLA-DRA, PTPN11, PTPN6, HLA-DRB1, LCK, CD4, and CD3E. When selecting PDGFRA as the target molecule, the target-related molecule is preferably selected from PDGFA, PDGFC, PIK3R1, STAT3, CRKL, CRK, PDGFB, STAT1, PTPN11, and GRB2. When selecting PD-L1 as the target molecule, the target-related molecule is preferably selected from PDCD1, PTPN11, PDCD1LG2, LCK, CD3E, CD4, HLA-DRB1, HLA-DPB1, HLA-DRA, and CD3G. When selecting VEGF as the target molecule, the target-related molecule is preferably selected from FLT1, KDR, HIF1A, FLT4, EGF, THBS1, IGF1, HGF, TGFB1, and PIK3CA. When selecting VEGFR as the target molecule, the target-related molecule is preferably selected from VEGFC, VEGFA, CDH5, FIGF, NRP1, FGF2, PIK3CA, PIK3CB, CRK, and NOS3.

### <Step (B)>

The step (B) in the present invention is a step of measuring blood concentration of the medicine X in a subject.

In addition, in the method for assessing medicine X of the present invention, the step (B) is performed in combination with the step (P) and at least one, preferably both, of the steps (A) and (C).

The measurement method is not particularly limited, and a general method can be used. More accurate blood concentration can be obtained by using a plasma separated from blood collected from a subject by centrifugation or the like as a specimen, and regarding the measured plasma concentration as a blood concentration.

When the medicine X is an antibody medicine, it is preferable to use ELISA.

When the medicine X is a nucleic acid medicine, it is preferable to use a hybridization method that utilizes a property that a nucleic acid forms a double strand with a complementary strand. In the case of a nucleic acid aptamer, the concentration is determined by hybridizing the nucleic acid aptamer using a sequence complementary to a half sequence of the nucleic acid aptamer as a fixing probe and the other half sequence as a detection probe.

### <Step (C)>

The step (C) in the present invention is a step of calculating a proportion of cells reached by the medicine X.

It is preferable that the calculation of a proportion of cells reached by the medicine X is performed by visualizing the medicine X taken into the tissue. For example, it is preferably performed by an immunostaining method using a phosphor integrated dot (PID), using a tissue section prepared from a tissue of a lesion part (e.g., tumor part) related to an adaptive disease of an antibody medicine as a specimen, collected from a subject to which the antibody medicine was administered. For example, the antibody medicine can be stained using a secondary antibody that specifically recognizes and binds to an antibody used as the antibody medicine, to which a PID directly or indirectly bound, and specifically, in the case where trastuzumab that is a humanized anti-HER2 antibody is used as the antibody medicine, trastuzumab in a tissue can be stained by performing staining using an anti-human IgG antibody, to which a PID is bound. Moreover, when a nucleic acid aptamer that recognizes human HER2 is used as a nucleic acid medicine, the antibody medicine can be stained using a nucleic acid probe having a sequence complementary to the nucleic acid aptamer, to which a PID is bound, or can be stained using the nucleic acid probe to which a first biological substance is bound and a second biological substance that specifically recognizes the first biological substance, to which a PID directly or indirectly bound.

Further, the specimen stained with the phosphor integrated dot (PID) is further stained for bright field observation, whereby the number of cells in the specimen can be measured together. The staining method for bright field observation is not particularly limited, but typically, hematoxylin-eosin staining (HE staining) is performed. When performing such bright field observation staining, the staining may be performed after an immunostaining process or may be performed before the immunostaining process. The number of cells may be measured digitally using software or the like, or may be measured visually. In this way, by measuring the number of trastuzumab-stained cells and the total number of cells contained in the whole specimen or in the unit area (or volume), a ratio of the number of cells from which a bright spot (cells reached by the antibody medicine) was observed to the total number of cells, that is, a proportion of cells reached by the antibody medicine can be calculated. Alternatively, the number of bright spots per cell in the cells reached by the antibody medicine may be calculated together.

### (Specimen)

The assessment method of the present invention is performed in vitro on a subject using the following specimen.

The specimen (which is referred to herein as specimen (i) in the present specification) used in the step (B) is derived from the blood of the subject to which the medicine X has been administered, and is preferably a plasma separated by centrifugation or scientific treatment of the blood of the subject collected according to a conventional method. The specimen (i) is preferably one that has been appropriately treated in accordance with a measurement method to be further performed in the step (B), and may contain an appropriate additive or the like for storage and optimization of measurement.

The specimen (which is referred to herein as specimen (ii)) used in the step (P), the step (A), and the specimen used in the step (C) (which is referred to herein as specimen (iii)) refer to a tissue section collected from the subject, or cells obtained by culturing cells contained in a tissue collected from the subject, and generally preferably take the form of a specimen slide on which the tissue section and cells are mounted, as is commonly used in cases for assessing expression level of a protein of interest by an immunostaining method, and the like. A specimen slide on which a tissue section is mounted herein is particularly called a tissue slide.

The specimen (ii) is derived from the subject before administration of medicine X.

The specimen (iii) is derived from the subject after administration of medicine X.

The specimen is preferably derived from a diseased tissue, especially derived from a tumor tissue. The "tumor tissue" is a tissue containing cancer cells collected from a subject according to a conventional method, and may contain, besides cancer cells, for example, normal cells, blood vessels, stromal cells (fibroblasts, endothelial cells, white blood cells (lymphocytes, monocytes, neutrophils, eosinophils, basophils), and the like), and the like.

The "tumor" is typically a solid tumor (cancer) and is not particularly limited, and examples thereof include solid cancers such as cytoma, melanoma, sarcoma, brain tumor, head and neck cancer, gastric cancer, lung cancer, breast cancer, liver cancer, colon cancer, cervical cancer, prostate cancer, and bladder cancer; leukemia; lymphomas; multiple myelomas; and the like.

The "specimen derived from a tumor tissue" is a tumor tissue section or a cancer cell mass collected from a lesion part of a subject having a tumor (or suspected of having a tumor), or cells obtained by culturing tumor cells contained in a tumor tissue collected from the subject, and preferably takes the form of a specimen slide as described above. When the "specimen derived from a tumor tissue" is a tumor tissue section, the specimen may contain surrounding normal tissue in addition to the tumor tissue. A region containing a tumor tissue (cancer cell) in a specimen derived from a tumor tissue is herein referred to as a "tumor region".

The method for preparing the tissue section and the specimen slide is not particularly limited, and generally, for example, a tissue sample prepared by fixing a tissue collected from a subject using formalin or the like, subjecting to dehydration with alcohol, followed by xylene treatment, and embedding in paraffin by immersing it in paraffin at high temperature is cut into 3 to 4 µm sections to obtain a tissue section, and a specimen slide (tissue slide) can be prepared by placing the tissue section on a slide glass and drying it.

### (Immunostaining)

As described above, the steps (P) and (A) in the present invention are preferably performed by an immunostaining method using a phosphor integrated dot (PID). When the medicine X is an antibody medicine, it is preferable that not only the step (P) and the step (A) but also the step (C) be performed by an immunostaining method using a phosphor integrated dot (PID). Hereinafter, as an example of an embodiment of the present invention, an example of an immunostaining method using PID will be described in further detail, using a specimen derived from a tumor tissue, specifically, a tissue slide prepared from a tumor tissue section.

### (1. Specimen slide pretreatment process)

### (1-1. Deparaffinization treatment)

A section is immersed in xylene put in a container to remove paraffin. The temperature is not particularly limited, but it can be performed at room temperature. The immersion time is preferably 3 minutes or more and 30 minutes or less. As needed, xylene may be replaced during immersion.

Subsequently, the section is immersed in ethanol put in a container to remove xylene. The temperature is not particularly limited, but it can be performed at room temperature. The immersion time is preferably 3 minutes or more and 30 minutes or less. As needed, ethanol may be replaced during immersion.

The section is immersed in water put in a container to remove ethanol. The temperature is not particularly limited, but it can be performed at room temperature. The immersion time is preferably 3 minutes or more and 30 minutes or less. As needed, water may be replaced during immersion.

### (1-2. Activation treatment)

Activation treatment of a substance of interest is performed according to a known method. Although activation conditions are not particularly limited, as an activation solution, a 0.01 M citrate buffer solution (pH 6.0), a 1 mM EDTA solution (pH 8.0), 5% urea, a 0.1 M tris hydrochloride buffer solution or the like can be used. As a heating device, an autoclave, a microwave, a pressure cooker, a water bath or the like can be used. The temperature is not particularly limited, but it can be performed at room temperature. The activation treatment can be performed at a temperature of 50 to 130°C for a time of 5 to 30 minutes.

Subsequently, the activated section is immersed in PBS put in a container and washed. The temperature is not particularly limited, but it can be performed at room temperature. The immersion time is preferably 3 minutes or more and 30 minutes or less. As needed, PBS may be replaced during immersion.

### (2. Immunostaining process)

In the immunostaining process, in order to stain a substance of interest (for example, a target molecule in the step (P)), a staining solution, in which a phosphor integrated dot having a site capable of directly or indirectly binding to the substance of interest is dispersed in a diluted solution, is placed on a tissue section and reacted with the substance of interest.

For example, in the case of an embodiment using a staining solution containing [avidin]-[phosphor integrated dot], first, a treatment of dropping a solution of a primary antibody onto a tissue slide, then, a treatment of dropping a solution of a secondary antibody-biotin conjugate onto a tissue section, and finally, a treatment of dropping the PID staining solution agent onto the tissue section may be performed. At this time, after the staining, the substance of interest and the phosphor integrated dot become [substance of interest]... [primary antibody against substance of interest]...[antibody (secondary antibody) against primary antibody]-[biotin]/[avidin]-[phosphor integrated dot] (where "..." indicates binding by an antigen-antibody reaction, "-" indicates binding by a covalent bond that may be via a linker molecule as needed, and "/" indicates binding by an avidin-biotin reaction).

Conditions under which the immunostaining process is performed, for example, temperature and time when the staining solution is dropped on the tissue slide to be reacted, washing time of the tissue slide and the like, can be appropriately adjusted to obtain appropriate signals according to the conventional immunostaining method.

The temperature is not particularly limited, but it can be performed at room temperature. The reaction time is preferably 30 minutes or more and 24 hours or less.

Prior to performing the primary reaction treatment as described above, it is preferable to drop a known blocking agent such as BSA-containing PBS or a surfactant such as Tween 20.

### (3. Specimen post-treatment process)

The tissue slide after the immunostaining process is preferably subjected to treatment such as fixation/dehydration, penetration and encapsulation, so as to be suitable for observation.

The fixation/dehydration treatment may be performed by immersing the tissue slide in a fixation treatment solution (a cross-linking agent such as formalin, paraformaldehyde, glutaraldehyde, acetone, ethanol or methanol). The penetration may be performed by immersing the tissue slide after the fixation/dehydration treatment in a penetration liquid (xylene or the like). The encapsulation treatment may be performed by dropping an encapsulation solution onto the tissue slide after the penetration treatment. Conditions under which these treatments are performed, for example, the temperature and immersion time when immersing the tissue slide in a predetermined treatment solution can be appropriately adjusted to obtain appropriate signals according to the conventional immunostaining method.

### (4. Optional process)

Even when the step (C) is not performed as an embodiment of the present invention, if necessary, staining for bright field observation can be performed. The staining method for bright field observation is not particularly limited, but typically, hematoxylin-eosin staining (HE staining) is performed. When the morphological observation staining process is included, it may be performed after the immunostaining process or may be performed before the immunostaining process.

### (5. Assessment process)

### (5-1. Observation and photographing)

In an observation and photographing process, a specimen sample is irradiated with excitation light corresponding to the phosphor integrated dots fluorescently labeling the substance of interest used in the immunostaining process thereto in the same field of view of a microscope at a desired magnification, and each of the immunostaining images by the fluorescence emitted from the phosphor integrated dots is observed and photographed. The excitation light can be irradiated by using, for example, a light source such as an ultra-high pressure mercury lamp, a Xe lamp, an LED (light emitting diode), a laser device or the like provided in a fluorescence microscope, and an optical filter for excitation light that selectively transmits a predetermined wavelength as needed. When immunostaining has been performed using a plurality of staining solutions containing different phosphor integrated dots, observation is performed while switching a plurality of types of filter sets corresponding to the respective phosphor integrated dots. The immunostaining images can be captured, for example, by a digital camera provided in a fluorescence microscope. In capturing of the immunostaining image, by using an optical filter for fluorescence that selectively transmits a predetermined wavelength as needed, it is possible to capture an immunostaining image which includes fluorescent of interest and which excludes fluorescent which is not of interest or excitation light noise and other types of light.

### (5-2. Image processing/signal measurement)

In an image processing/measurement process, with respect to an immunostaining image captured for the substance of interest, based on the image processing, a fluorescent labeling signal corresponding to a fluorescent labeling signal corresponding to the substance of interest is measured to identify a fluorescent labeling signal corresponding to the biological substance of interest within a region of a cell membrane. It is preferable to treat the fluorescent labeling signal as the number of fluorescent bright spots.

Examples of software that can be used for image processing include "ImageJ" (open source). By using such image processing software, extraction of bright spots of a predetermined wavelength (color) from the immunostaining image to calculate the total sum of luminance of the bright spots, measurement of the number of bright spots having predetermined luminance or more, a process of superimposing a plurality of images, a process of measuring the number of cells included in the images and the like can be semi-automatically and quickly performed.

In addition, since a bright spot is derived from one phosphor integrated dot, the bright spot has a constant size and can be recognized by microscopic observation. A signal larger than a fixed value (e.g., an average value of observed phosphor integrated dots) is determined as an aggregated bright spot. The bright spots and the aggregated bright spots can be semi-automatically and quickly distinguished using software.

### (Antibody)

When performing the immunostaining as described above in each step of the assessment method of the present invention, a monoclonal antibody is preferably used from the viewpoint of specificity. The type of animal (immunized animal) that produces the antibody is not particularly limited, and may be selected from mice, rats, guinea pigs, rabbits, goats, sheep, and the like, as in the conventional case.

Moreover, the antibody may not be a natural full-length antibody, but may be an antibody fragment or a derivative as long as it has an ability to specifically recognize and bind to a substance of interest for detection (for example, a target protein in the step (P)).

### (Tissue staining when medicine X is nucleic acid medicine)

When the medicine X is a nucleic acid medicine, the step (C) in the present invention is preferably performed by a staining method (FISH) using a nucleic acid probe reagent. Hereinafter, as an example of an embodiment of the present invention, an example of a staining method using a nucleic acid probe reagent will be described in further detail, using a specimen derived from a tumor tissue, specifically, a tissue slide prepared from a tumor tissue section. However, the staining method is not particularly limited, and a known method can be used.

### (1. Preparation of nucleic acid probe)

As long as it is a nucleic acid molecule having a sequence complementary to a nucleic acid medicine of interest, it may be DNA or RNA, and the length of the sequence can be also appropriately selected so as to hybridize efficiently.

### (2. Preparation of nucleic acid probe to which first biomolecule is bound)

As a method for binding a first biomolecule to a nucleic acid molecule, a PCR labeling method, a nick translation method, and a random prime method using a nucleic acid substrate having the first biomolecule can be used. In the PCR labeling method, a nucleic acid molecule can be labeled with a first biomolecule (e.g., biotin or the like) by performing PCR using a nucleic acid substrate (e.g., dUTP-biotin or the like) labeled with the first biomolecule using the nucleic acid molecule as a template. In the nick translation method, a nucleic acid molecule can be labeled with a first biomolecule by performing nick translation using a nucleic acid substrate (e.g., dUTP-biotin or the like) labeled with the first biomolecule. Alternatively, in the random prime method, a nucleic acid molecule can be labeled with a first biomolecule by forming primers having different lengths into double strands on a nucleic acid molecule serving as a template, and acting a Klenow enzyme thereon in the presence of a nucleic acid substrate (e.g., dUTP-biotin) labeled with the first biomolecule or the like.

Examples of the first biomolecule include haptens such as biotin, FITC, Cy5, DNP, DIG and the like which are small molecules.

### (3. Deparaffinization treatment process)

A tissue section on a specimen slide is immersed in xylene or other deparaffinizing agent put in a container to remove paraffin. The temperature at this time is not particularly limited, but it can be performed at room temperature. The immersion time is preferably 3 minutes or more and 30 minutes or less. As needed, xylene may be replaced during immersion. Subsequently, the section is immersed in ethanol put in a container to remove xylene. The temperature is not particularly limited, but it can be performed at room temperature. The immersion time is preferably 3 minutes or more and 30 minutes or less. As needed, ethanol may be replaced during immersion. Subsequently, the section is immersed in water put in a container to remove ethanol. The temperature is not particularly limited, but it can be performed at room temperature. The immersion time is preferably 3 minutes or more and 30 minutes or less. As needed, water may be replaced during immersion.

### (4.Pretreatment of specimen slide)

It has been known that, before subjecting the probe to a hybridization reaction, pretreatment is performed so that a probe reagent can efficiently reach the nucleic acid on the tissue section, such as pretreatment (heat treatment, acid treatment) or treatment with enzyme treatment. These treatment conditions and combinations have different optimal conditions depending on the type, thickness, slide adjustment conditions and the like of the section. Therefore, it is necessary to appropriately determine the procedure. Not all treatments need to be performed; for example, there may be an option not to perform enzyme treatment.

First, according to a known method, a specimen slide to be subjected to FISH is pre-treated. Although there is no particular limitation on the pretreatment conditions, for example, the pretreatment can be performed in the following procedure. First, a specimen slide is immersed in hydrochloric acid (about 0.2 mol/L) for a certain time. Thereafter, the specimen slide is immersed in water and further immersed in a washing buffer (2 × SSC: standard sailine citrate) for washing. Next, the specimen slide is immersed in a heated NaSCN solution (for example, about 1 N) for a certain time. Thereafter, the specimen slide is immersed in water and further immersed in a washing buffer for washing, and the same operation is repeated twice. Also, pretreatment can be also performed under heating, using a 0.01 M citrate buffer (pH 6.0), a 1 mM EDTA solution (pH 8.0), 5% urea, a 0.1 M Tris-HCl buffer or the like, instead of the above two types of solutions, as a pretreatment solution. As a heating device, an autoclave, a microwave, a pressure cooker, a water bath or the like can be used. The temperature is not particularly limited, but it can be performed at a temperature of 50 to 130°C for a time of 5 minutes or more and 30 minutes or less.

Subsequently, using a hydrolase (protease), an enzyme treatment for removing proteins, particularly collagen, of a cell membrane and a nuclear membrane is performed, for example, by the following procedure.

First, the specimen slide is immersed in a protease solution for a certain time. Subsequently, the specimen slide is immersed in a washing buffer for washing, and this operation is repeated twice.

Thereafter, the specimen slide is dried by air drying or the like. A known dehydration treatment using 70% to 100% ethanol may be performed instead of air drying.

As the protease, a proteinase suitable for protein hydrolysis, for example, pepsin, proteinase K and the like are often used. In addition, setting of an efficiency of deproteinization is performed in condition setting that does not impair morphological details, after examining hybridization, that is, a combination of protease concentration and degradation time to maximize a reaction with chromosome of interest. The optimum conditions differ depending on tissue type and fixation method. Moreover, additional fixation after protease treatment is useful.

If necessary, after the above enzyme treatment, in each step of the pretreatment, for example, the following procedure is performed to fix the specimen slide. First, a specimen slide is immersed in a formalin solution for a certain time. Subsequently, the specimen slide is immersed in a washing buffer for washing, and this operation is repeated twice. Thereafter, the specimen slide is dried by air drying or the like.

In addition, usually, staining is performed without performing any fixation treatment, and a fixation treatment is added as a process implemented for improvement after the process where the section is considered to have been peeled off. Therefore, the fixation treatment may not be implemented at all in some cases, and may be implemented in several positions in other cases. Therefore, the fixation treatment may be performed before and after the nuclear staining as needed, even in steps other than each step other than the pretreatment. For example, the specimen may be fixed before and/or after the hybridization.

### (5. Staining process)

### (5-1. DNA denaturation treatment)

After the above fixation treatment, in order to denature DNA present on the section (from double-stranded DNA to single-stranded DNA), for example, the following procedure is performed. First, the specimen slide is immersed in a denaturing solution (formamide/SSC solution or the like) at about 72°C for a predetermined time. Thereafter, the specimen slide is taken out and immersed in several stages of ethanol (for example, 70% aqueous ethanol solution, 80% aqueous ethanol solution, and 100% ethanol) in which the concentration is gradually increased to remove formamide. Thereafter, the specimen slide is dried by air drying or the like.

### (5-2. Hybridization treatment)

Using a probe reagent containing a nucleic acid molecule (nucleic acid probe) having a sequence complementary to the nucleic acid medicine of interest, hybridization treatment can be performed in the same manner as in a known FISH method (for example, "Agilent FISH General PurposeReagents Protocol" or "Clinical FISH Protocol-Visualization of Chromosomal/Genetic Diagnosis (Cell Engineering Supplement-Experimental Protocol Series")). The term "hybridization" used herein means a process of binding two DNAs or a DNA and an RNA complementary strand for the formation of a double-stranded molecule, or a formed double-stranded molecule formed.

One embodiment of the above hybridization is hybridization in which, first, a probe reagent containing a complex in which a nucleic acid probe and a phosphor integrated dot are bound is prepared, and the complex is bound to each nucleic acid medicine of interest.

Another embodiment of the above hybridization is dynamic hybridization via an antigen-antibody reaction, in which a probe reagent separately having a nucleic acid probe solution and a phosphor integrated dot dispersion was prepared, and only the nucleic acid probe was first hybridized to the nucleic acid medicine of interest, then an anti-hapten antibody is bound to hapten which is the first biomolecule bound to the hybridized nucleic acid probe, and further, a third biomolecule bound to a fluorescent nanoparticle is bound to a second biomolecule bound to the antibody. The second biomolecule can be selected from haptens as well as the first biomolecule, and it is not preferable to select the same hapten. Examples of the third biomolecule include strepavidin, avidin, neutravidin and the like, which are macromolecules. Among them, streptavidin can be suitably used.

The type of nucleic acid molecule used for hybridization is not limited to one type, and may be two or more types.

In addition, as long as the phosphor integrated dots can be respectively bound to the nucleic acid probe forming a complementary strand with the nucleic acid medicine of interest, another mode of hybridization may be used. The nucleic acid molecule may be directly or dynamically bound to the phosphor integrated dots, for example, by using each binding (a binding between a hapten and an anti-hapten antibody, a binding between a first biomolecule and a second biomolecule of the probe, a binding between a first binding group and a second binding group), or a combination of these bindings.

### (5-3. Nuclear staining treatment)

After the hybridization treatment, usually, a nuclear staining treatment for counting the number of cells is further performed. DAPI is generally used as the nuclear staining reagent, but other than this, bisbenzimide derivatives such as Hoechst 33258 and Hoechst 33342 and other nuclear staining reagents may be used. For example, when DAPI is used as a nuclear staining reagent, nuclear staining can be performed in the following procedure. First, the specimen slide subjected to the hybridization treatment is sequentially washed with deionized water and phosphate buffered saline (PBS). Subsequently, the specimen slide is immersed in a DAPI staining reagent (2 µg/PBS) for a certain time.

### (5-4. Encapsulation treatment)

The specimen slide after the staining treatment by FISH and the nuclear staining treatment are washed several times with PBS, air-dried or dehydrated, and then encapsulation treatment in which an encapsulating agent is dropped on the tissue section, and the specimen slide is covered with a cover glass, and dried is performed. As the encapsulating agent, a known oil-based encapsulating agent (such as Entellan (registered trademark) new) or an aqueous encapsulating agent (such as Aquatex (registered trademark)) can be used. The encapsulated specimen slide prepared by the above treatment serves as a preparation for performing a pathological diagnosis or the like.

### (6. Observation process)

### (6-1. Bright field observation)

The bright field observation is performed as needed to obtain distribution information of cells or organs to be stained in cells or tissues. As a general method for bright field observation, for example, it is preferable to observe with a microscope after performing the above staining, performing hematoxylin-eosin staining (HE staining), or performing DAPI staining as described above.

When HE staining is performed, for example, immunostained sections are stained with Mayer's hematoxylin solution for 5 minutes to perform hematoxylin staining, and then, the tissue sample is washed with running water at 45°C for 3 minutes, and subsequently stained with 1% eosin solution for 5 minutes to perform eosin staining.

In addition, eosin used for morphological observation staining can not only be observed in a bright field, but also emits autofluorescence when irradiated with excitation light of a predetermined wavelength, so that eosin can also be observed with a fluorescence microscope by irradiating the stained tissue sample with excitation light of an appropriate wavelength and output.

On the other hand, as the other staining, for example, in bright field observation when histochemical staining (DAB staining or the like) is performed using HER2 protein in breast cancer as an antigen to be detected, a HER2 protein-positive staining image, intensity of the positive staining, and a proportion of positive cells of cancer cells in the specimen tissue are observed, using a 4 × objective lens of an optical microscope, under appropriate illumination light. Next, the objective lens is switched to 10 times, and whether a positive finding is localized in the cell membrane or cytoplasm is confirmed, and as needed, a search is further performed with an objective lens at 20 times.

### (6-2.Fluorescence observation)

This can be performed in the same manner as (5-2. Image processing/signal measurement) in (Immunostaining).

### (Phosphor integrated dot)

The phosphor integrated dot (PID) used in the present invention is a nano-sized dot having a structure, using a dot made of an organic or inorganic substance as a matrix, in which a plurality of phosphors are integrated inside (contained in) the matrix and/or are adsorbed on the matrix surface. When the phosphor is contained in the matrix, the phosphor only needs to be dispersed inside the matrix, and may or may not be chemically bound to the matrix itself. For example, the matrix (for example, resin) and the phosphor may be bound by electrostatic interaction or may be covalently bonded.

Being irradiated with an electromagnetic wave (X-ray, UV ray, or visible ray) having a predetermined wavelength, the "phosphor" herein absorbs the energy of the electromagnetic wave to excite electrons and emits extra energy as an electromagnetic wave when returning from the excited state to the ground state, in short, the "phosphor" is a substance emitting "fluorescence", representing a substance that directly or indirectly binds to a biological substance-recognizing substance (a substance that can specifically recognize a biological substance, e.g., biotin, avidin, an antibody, or the like). In addition, the "fluorescent" has a broad meaning, including narrowly-defined fluorescence with a short emission lifetime and phosphorescence with a long emission lifetime which enables duration of light emission even when irradiation of an electromagnetic wave for excitation is stopped.

Among the matrixes that form the phosphor integrated dot, examples of organic matters include resins generally classified as thermosetting resins such as melamine resin, urea resin, aniline resin, guanamine resin, phenol resin, xylene resin, and furan resin; resins generally classified as thermoplastic resins such as styrene resin, acrylic resin, acrylonitrile resin, AS resin (acrylonitrile-styrene copolymer), and ASA resin (acrylonitrile-styrene-methyl acrylate copolymer); other resins such as polylactic acid; and polysaccharides, and examples of inorganic matters include silica and glass.

Semiconductor nanoparticles and organic fluorochromes are preferably used as the phosphor. Examples of the semiconductor nanoparticles include those containing a II-VI compound, a III-V compound, or a group IV element, and specifically, CdSe, CdS, CdTe, ZnSe, ZnS, ZnTe, InP, InN, InAs, InGaP, GaP, GaAs, Si, Ge, and the like. Examples of the fluorochrome include rhodamine-based dye molecules, squarylium-based dye molecules, cyanine-based dye molecules, aromatic ring-based dye molecules, oxazine-based dye molecules, carbopironine-based dye molecules, and pyromecene-based dye molecules, and Alexa Fluor (registered trademark, manufactured by Invitrogen) dye, BODIPY (registered trademark, manufactured by Invitrogen) dye, Cy (registered trademark, manufactured by GE Healthcare) dye, DY (registered trademark, manufactured by DYOMICS GmbH) dye, HiLyte (registered trademark, manufactured by AnaSpec, Inc.) dye, DyLight (registered trademark, manufactured by Thermo Scientific) dye, ATTO (registered trademark, manufactured by ATTO-TEC GmbH) dye, MFP (registered trademark, manufactured by Mobitec) dye or the like can be used. In addition, the generic terms of such dye molecules are named based on the main structure (skeleton) of compounds or based on the registered trademarks, and those skilled in the art may properly understand which fluorochrome belongs to which dye molecule without excessive trial and error.

Other examples of the phosphor to be contained in the phosphor integrated dot include "high-persistence phosphors" that contain Y₂O₃, Zn₂SiO₄ or the like as a main component and Mn²⁺, Eu³⁺ or the like as an activator agent.

A phosphor integrated dot can be produced according to a known method (see, for example, JP 2013 - 57937 A).

More specifically, for example, a phosphor integrated silica particle using silica as a matrix, in which the phosphor is integrated in the matrix, can be prepared by dropping a solution in which a phosphor such as inorganic semiconductor nanoparticles and organic fluorochrome and a silica precursor such as tetraethoxysilane is dissolved into a solution in which ethanol and ammonia are dissolved so as to hydrolyze the silica precursor.

On the other hand, with regard to a phosphor integrated resin particle that includes resin as the matrix and phosphor adsorbed on a surface of the resin particle or contained therein, such a resin particle can be prepared by procuring a solution of the resin or a dispersion of fine particles in advance, adding a phosphor such as inorganic semiconductor nanoparticles or organic fluorochromes thereto, and stirring the mixture. Alternatively, a resin particle containing a phosphor can be prepared by proceeding a polymerization reaction after adding fluorochromes to a solution of a resin raw material. For example, when a thermosetting resin such as melamine resin is used as the matrix resin, an organic fluorochrome-containing resin particle can be prepared by heating a reaction mixture containing a raw material of the resin (monomer, oligomer, or prepolymer: for example, methylolmelamine which is a condensate of melamine and formaldehyde), organic fluorochromes, and preferably, a surfactant and a polymerization reaction accelerator (such as an acid), and proceeding a polymerization reaction by emulsion polymerization. When a thermoplastic resin such as a styrene copolymer is used as the matrix resin, an organic fluorochrome-containing resin particle can be prepared by heating a reaction mixture containing a raw material of the resin, organic fluorochromes (as a raw material monomer of the resin, a monomer to which organic fluorochromes are linked by a covalent bond may be used), and a polymerization initiator (such as benzoyl peroxide and azobisisobutyronitrile), and proceeding a polymerization reaction by radical polymerization or ion polymerization.

The average particle size of the phosphor integrated dots is not particularly limited as long as it is a particle size suitable for immunostaining of a specimen slide. However, in consideration of the ease of detection as a bright spot, the average particle size is usually 10 to 500 nm, and preferably 50 to 200 nm. Further, the coefficient of variation indicating the variation of the particle diameter is usually 20% or less, and preferably 5 to 15%. The phosphor integrated dot satisfying such conditions can be produced by adjusting production conditions. For example, when preparing a phosphor integrated dot by an emulsion polymerization method, the particle size can be adjusted by the amount of surfactant to be added. In general, when the amount of surfactant relative to the amount of matrix raw material of the phosphor integrated dot is relatively large, the particle size tends to be small, and when the amount is relatively small, the particle size tends to be large.

With regard to the particle diameter of the phosphor integrated dot, a cross-sectional area of the phosphor integrated dots is measured by taking an electron micrograph using a scanning electron microscope (SEM), and assuming that the cross-sectional shape is a circle, the particle diameter can be calculated as the diameter of a circle corresponding to the cross-sectional area. With regard to the average particle size and the coefficient of variation of a population of a plurality of phosphor integrated dots, the particle sizes for a sufficient number (for example, 1000) of phosphor integrated dots are calculated as described above, then the average particle diameter is calculated as an arithmetic average of those sizes, and the coefficient of variation is calculated according to the following expression: 100 × standard deviation of particle size/average particle size.

### Examples

Hereinafter, preferred embodiments of the present invention will be described more specifically with reference to Examples, but the present invention is not limited to these Examples.

### [Preparation Example 1]

### (Preparation of Biotin-Modified IgG Antibody)

In a 50 mM Tris solution, 50 µg of an anti-rabbit IgG antibody (Gene Tex: GTX40383) used as a secondary antibody was dissolved. A DTT (dithiothreitol) solution was added to this solution and mixed to a final concentration of 3 mM, and allowed to react at 37°C for 30 minutes. Thereafter, the reaction solution was passed through a desalting column "Zeba Desalt Spin Columns" (Thermo Scientific, Cat. #89882) to purify the secondary antibody reduced with DTT. 200 µL of the total amount of the purified antibody was dissolved in a 50 mM Tris solution to prepare an antibody solution. On the other hand, the linker reagent "Maleimide-PEG2-Biotin" (Thermo Scientific, product number 21901) was adjusted to 0.4 mM using DMSO. 8.5 µL of this linker reagent solution was added to the antibody solution and mixed, and allowed to react at 37°C for 30 minutes, thereby binding biotin to the anti-rabbit IgG antibody via a PEG chain. The reaction solution was purified by passing through a desalting column. For the desalted reaction solution, by measuring the absorbance at a wavelength of 300 nm using a spectrophotometer ("F-7000" manufactured by Hitachi, Ltd.), the concentration of protein (biotin-modified IgG antibody) in the reaction solution was calculated, and the concentration of the biotin-modified anti-rabbit IgG antibody was adjusted to 250 µg/mL using a 50 mM Tris solution.

In addition, a biotin-modified anti-mouse IgG antibody was prepared in the same manner as described above, except that an anti-mouse IgG antibody (Abcam: ab99601) was used in place of the anti-rabbit IgG antibody, and the concentration of the biotin-modified anti-mouse IgG antibody was adjusted to 250 µg/mL.

### [Preparation Example 2]

After dissolving 2.5 mg of Texas Red dye molecule "Sulforhodamine 101" (Sigma-Aldrich) in 22.5 mL of pure water, the solution was stirred with a hot stirrer for 20 minutes while the temperature of the solution was maintained at 70°C. To the stirred solution, 1.5 g of melamine resin "Nicalac MX-035" (Nippon Carbide Industries Ltd.) was added, and the mixture was further heated and stirred for 5 minutes under the same conditions. To the stirred solution, 100 µL of formic acid was added, and the mixture was stirred for 20 minutes while the temperature of the solution was maintained at 60°C, then the solution was allowed to stand to cool to room temperature. The cooled solution was dispensed into a plurality of centrifugation tubes, and centrifuged at 12,000 rpm for 20 minutes to precipitate Texas Red dye-containing melamine resin particles contained in the solution as a mixture. Supernatants were removed and precipitated particles were washed with ethanol and water. When SEM observation was performed on 1,000 resin particles obtained and the average particle diameter was measured as described above, the average particle diameter was 152 nm. The Texas Red dye-containing melamine resin particles thus prepared was surface-modified by the following procedure.

In 1.5 mL of EtOH, 0.1 mg of the obtained particles were dispersed, and 2 µL of aminepropyltrimethoxysilane LS-3150 (manufactured by Shin-Etsu Chemical Co., Ltd.) was added thereto, and allowed to react for 8 hours to perform a surface amination treatment.

Subsequently, the particles subjected to the surface amination treatment were adjusted to 3 nM using PBS (phosphate buffered saline) containing 2 mM EDTA (ethylenediaminetetraacetic acid), SM (PEG) ₁₂ (succinimidyl-[(N-maleimidopropionamido)-dodecaethyleneglycol]ester manufactured by Thermo Scientific) was mixed to the solution to a final concentration of 10 mM, and allowed to react for 1 hour. The mixture was centrifuged at 10,000 G for 20 minutes, and the supernatant was removed. Then, PBS containing 2 mM EDTA was added thereto to disperse precipitates, and the mixture was centrifuged again. Washing was performed three times by the same procedure to obtain maleimide-modified Texas Red-integrated melamine particles.

On the other hand, streptavidin (manufactured by Wako Pure Chemical Industries, Ltd.) was subjected to a thiol group addition treatment using N-succinimidyl S-acetylthioacetate (SATA), followed by filtration through a gel filtration column to obtain thiol-modified streptavidin.

The maleimide-modified Texas Red-integrated melamine particles and thiol-modified streptavidin were mixed in PBS containing 2 mM EDTA, and allowed to react at room temperature for 1 hour. The reaction was stopped by adding 10 mM mercaptoethanol. After concentrating the obtained solution with a centrifugal filter, unreacted streptavidin and the like were removed using a gel filtration column for purification, and the obtained streptavidin-bound Texas Red-integrated melamine particles were collected. The obtained streptavidin-bound Texas Red-integrated melamine particles diluted to 0.06 nM with a diluent for phosphor integrated dots were used as a red PID staining solution in Experimental Examples 2 and 3.

### [Preparation Example 3]

According to the procedure described in Preparation Example 2, FITC was used in place of Texas Red dye molecule "Sulforhodamine 101" (Sigma-Aldrich) to prepare FITC dye-integrated melamine resin particles. The obtained streptavidin-bound FITC-integrated melamine resin particles diluted to 0.06 nM with a diluent for phosphor integrated dots were used as a green PID staining solution in Experimental Examples 2 and 3.

### [Preparation Example 4]

According to the procedure described in Preparation Example 2, 7-(Diethylamino)-3-Phenylcoumarin was used in place of Texas Red dye molecule "Sulforhodamine 101" (Sigma-Aldrich) to prepare 7-(Diethylamino)-3-Phenylcoumarin dye-integrated melamine resin particles. 7-(Diethylamino)-3-Phenylcoumarin dye-integrated melamine resin particles that were maleimide-modified in the same manner as in Preparation Example 1, and anti-trastuzumab antibody (Bio-rad; HCA177) that was thiol-modified in place of streptavidin in the same manner as in Preparation Example 1, were reacted and treated in the same manner as in Preparation Example 1 to prepare anti-trastuzumab antibody-bound 7-(Diethylamino)-3-Phenylcoumarin dye-integrated melamine resin particles, and those diluted to 0.06 nM with a diluent for phosphor integrated dots were used as a blue PID staining solution in Experimental Example 2.

### [Preparation Example 5]

According to the procedure described in Preparation Example 2, 7-(Diethylamino)-3-Phenylcoumarin was used in place of Texas Red dye molecule "Sulforhodamine 101" (Sigma-Aldrich) to prepare 7-(Diethylamino)-3-Phenylcoumarin dye-integrated melamine resin particles. The obtained streptavidin-bound 7-(Diethylamino)-3-Phenylcoumarin-integrated melamine resin particles diluted to 0.06 nM with a diluent for phosphor integrated dots were used as a blue PID staining solution in Experimental Example 4.

### <Assessment Preparation 1>

Tumor tissues collected from multiple breast cancer patients were purchased, and 2 mm-square tumor tissues corresponding to each patient were implanted subcutaneously in the lumbar region of acquired immunodeficient mice, whereby preparing PDX model mice. When the tumor tissue grew to about 300 mm³ (a month after implantation), administration of trastuzumab (trade name; Herceptin (registered trademark)) was started. The administration conditions were: a dose of 30 mg/kg; a number of doses of once a day, twice a week.

The following steps were performed for each of the mice before and after the administration. As a control mouse, a PDX mouse corresponding to each patient that is administered with PBS in place of trastuzumab under the same conditions is used.
(I) From the mouse 40 days after the administration, blood was collected using an injection needle (Termo Corporation) which sucked up heparin Na injection 10,000 units/10 mL "Mochida" (Mochida Pharmaceutical Co., Ltd.) and then ejected it, and the plasma was separated by centrifugation at 1200 g for 20 minutes. The plasma was diluted 100-fold using 0.3% BSA/0.05% tween 20/140 mM NaCl/25 mM sodium phosphate (pH 7.2) as a diluent.
(II) Immediately before the first administration, a part of the tumor was collected by needle biopsy, and several FFPE (formalin-fixed paraffin-embedded) tissue slides were prepared for each mouse.
(III) 40 days after the administration, a part of the tumor was collected by needle biopsy, and several FFPE (formalin-fixed paraffin-embedded) tissue slides were prepared for each mouse.
(IV) The tumor volume was measured immediately before the first administration and 40 days after the administration.

### [Experimental Example 1]

Using the diluted plasma obtained in step (I) as a specimen, a test was performed using Anti-Trastuzumab, ELISA Kit, and ADA ELISA (somrubioscience) to measure the blood concentration of trastuzumab. Fig. 1 shows a diagram in which the blood concentration of the antibody medicine for each specimen is plotted. It can be seen that each specimen can be classified into a high concentration group and a low concentration group based on the trastuzumab blood concentration.

### [Experimental Example 2]

The tissue slides prepared in the above steps (II) and (III) were subjected to immunostaining according to the following procedure.

### (Pretreatment of specimen)

After the prepared FFPE tissue slide was deparaffinized, washing was performed by replacing with water. The washed tissue slide was heated in a 10 mM citrate buffer (pH 6.0) at 95°C for 40 minutes to activate an antigen. The activated tissue slide was washed with PBS. A diluent for fluorescent nanoparticles (a solution containing casein (composition = α-casein: 50 w/w%, β-casein BSA: 50 w/w%) and 1% BSA) was dropped on the washed tissue slide, thereby performing a blocking treatment for 15 minutes.

### (Immunostaining A)

For the tissue slide prepared in step (II), PBS prepared to contain anti-HER2 rabbit monoclonal antibody (Roche; 4B5) at 5 µg/µL was dropped on the tissue slide subjected to the specimen pretreatment process, and allowed to react at 4°C overnight, then the tissue slide was washed with PBS. The biotin-modified anti-rabbit IgG antibody prepared in Preparation Example 1 further diluted to 2 µg/mL with a diluent for phosphor integrated dots was dropped on the washed tissue slide, and allowed to react at room temperature for 30 minutes. After the tissue slide was further washed with PBS, the red PID staining solution prepared in Preparation Example 2 was dropped thereon and allowed to react at room temperature for 2 hours.

After streptavidin-biotin block treatment, PBS prepared to contain anti-HER3 antibody mouse monoclonal antibody (Novus Biologicals; RTJ2) at 5 µg/µL was dropped on the tissue slide, and allowed to react at 4°C overnight, then the tissue slide was washed with PBS. The biotin-modified anti-mouse IgG antibody prepared in Preparation Example 1 further diluted to 2 µg/mL with a diluent for phosphor integrated dots was dropped on the washed tissue slide, and allowed to react at room temperature for 30 minutes. After the tissue slide was further washed with PBS, the green PID staining solution prepared in Preparation Example 2 was dropped thereon and allowed to react at room temperature for 2 hours.

### (Immunostaining B)

For the tissue slide prepared in step (III), the blue PID staining solution prepared in Preparation Example 3 was dropped on the tissue slide subjected to the specimen pretreatment process, and allowed to react at 4°C overnight.

### (Staining treatment for morphological observation)

The tissue slide subjected to the immunostaining A or B was stained with Mayer's hematoxylin solution for 5 minutes to perform hematoxylin staining, and then washed with running water for 10 minutes.

### (Posttreatment of specimen)

The tissue slide that had been subjected to immunostaining was subjected to a fixation and dehydration treatment in which an operation of immersing the tissue slide in pure ethanol was performed four times. Subsequently, the tissue slide was subjected to a penetration treatment in which an operation of immersing in xylene was performed three times. Finally, an encapsulating agent "Marinol" (MUTO PURE CHEMICALS CO.,LTD.) was placed thereon, and an encapsulation treatment of covering with a cover glass was performed to prepare a specimen used for observation.

### (Observation, photographing, and image processing)

The number of fluorescent bright spots was measured, with respect to Texas Red used for fluorescent labeling of HER2 and FIFC used for fluorescent labeling of HER3 for the tissue slide prepared in the step (II), and with respect to 7-(Diethylamino)-3-Phenylcoumarin used for fluorescent labeling of trastuzumab for the tissue slide prepared in the step (III).

A fluorescence microscope "BX-63" (Olympus Corporation) was used to observe fluorescence, and a digital microscope camera "DP80" (Olympus Corporation) attached to the fluorescence microscope was used to capture fluorescence images (400 ×), at five fields of view per tissue slide.

For the tissue slide prepared in the step (II), first, the specimen was irradiated with excitation light corresponding to Texas Red used for fluorescent labeling of HER2 to emit fluorescence, an immunostaining image in the state was captured, and the number of bright spots representing the Texas Red-integrated melamine particles having a luminance of a predetermined value or more was measured. At this time, the wavelength of the excitation light was set to 575 to 600 nm using an optical filter for excitation light provided in the fluorescence microscope, and the wavelength of fluorescence to be observed was set to 612 to 692 nm using an optical filter for fluorescence. Next, the specimen was irradiated with excitation light corresponding to FIFC used for the fluorescent labeling of HER3 to emit fluorescence, a fluorescent image in that state was captured, and the number of bright spots representing the FIFC-integrated melamine particles having a luminance of a predetermined value or more was measured.

At this time, the wavelength of the excitation light was set to 470 to 495 nm using an optical filter for excitation light provided in the fluorescence microscope, and the wavelength of fluorescence to be observed was set to 510 to 550 nm using an optical filter for fluorescence.

For the tissue slide prepared in the step (III), the specimen was irradiated with excitation light corresponding to Phenylcoumarin used for fluorescent labeling of trastuzumab to emit fluorescence, a fluorescence image in the state was captured, and the number of bright spots representing the integrated melamine particles having a luminance of a predetermined value or more was measured. At this time, the wavelength of the excitation light was set to 340 to 390 nm using an optical filter for excitation light provided in the fluorescence microscope, and the wavelength of fluorescence to be observed was set to 420 to 460 nm using an optical filter for fluorescence.

The intensity of the excitation light during observation and imaging with the fluorescence microscope was both adjusted in such a manner that irradiation energy around the center of a field of view became to 900 W/cm², and the exposure time during imaging was set in a range that made the luminance of each image unsaturated, for example, 4000 µseconds.

In the same visual field where the fluorescence observation and the imaging of the fluorescent image were performed, bright-field observation and imaging were performed to capture staining images obtained by the hematoxylin staining.

The obtained fluorescence image and bright field image were superimposed by image processing, and the number of cells contained in each image and the bright spots indicating the phosphor integrated dots labeled with HER2, HER3 or trastuzumab were measured, respectively. Further, the ratio of the number of cells including the bright spot indicating the phosphor integrated dot labeled with HER2 and HER3 or trastuzumab to the total number of cells included in the image was calculated.

For image processing in this process, image processing software "Image J" (open source) was used.

### (Assessment process)

Table 1 shows the results of Experimental Examples 1 and 2 for each specimen. Cells including a bright spot indicating a HER2-labeled phosphor integrated dot are referred to as HER2 + cells, cells including a bright spot indicating a HER3-labeled phosphor integrated dot are referred to as HER3 + cells, and cells including a bright spot indicating a trastuzumab-labeled phosphor integrated dot are referred to as cells reached by drug.

In addition, a graph obtained by dividing the specimens into a high-concentration group and a low-concentration group based on the trastuzumab blood concentration in Experimental Example 1, further dividing the high-concentration group into a HER3 high expression group and a HER3 low expression group, and plotting a ratio of HER2 + cells and a proportion of cells reached by trastuzumab in each group is shown in Fig. 2.

**[Table 1]**

| | Agent blood concentration (µg/mL) | HER2 + cells (%) | HER3 + cells (%) | Proportion of cells reached by agent (%) |
|---|---|---|---|---|
| Specimen A | 570 | 0 | 0 | 0 |
| Specimen B | 650 | 30 | 5 | 25 |
| Specimen C | 580 | 60 | 0 | 50 |
| Specimen D | 500 | 100 | 5 | 80 |
| Specimen E | 470 | 0 | 80 | 0 |
| Specimen F | 570 | 30 | 70 | 10 |
| Specimen G | 620 | 60 | 70 | 25 |
| Specimen H | 540 | 100 | 80 | 40 |
| Specimen I | 320 | 0 | 5 | 0 |
| Specimen J | 310 | 30 | 5 | 15 |
| Specimen K | 210 | 60 | 0 | 20 |
| Specimen L | 300 | 100 | 0 | 50 |

### [Experimental Example 3]

From the tumor volume immediately before the first administration and the tumor volume 40 days after the administration measured in the step (IV), each of the ratio of the tumor volume 40 days after the administration to the tumor volume immediately before the first administration was calculated, and the tumor volume increase rate was calculated.

The tumor volume increase rate was compared with the tumor volume increase rate of a control group (control mouse) derived from the same patient, respectively, and the ratio was calculated. The therapeutic effect of trastuzumab was assessed using a 4-point treatment score based on the following criteria.

On 40 days after the administration, one in which complete tumor regression was observed was rated 3, one with a tumor volume increase rate of less than 50% of the control group was rated 2, one with a tumor volume increase rate of 50% or more and less than 75% of the control group was rated 1, and one with a tumor volume increase rate of 75% to 100% of the control group was rated 0. Table 2 shows treatment scores of each specimen together with the results of Experimental Examples 1 and 2.

Moreover, Fig. 3 shows a graph in which the horizontal axis represents the proportion of cells reached by trastuzumab calculated in Experimental Example 2, and the vertical axis represents the therapeutic effect of trastuzumab.

**[Table 2]**

| | Agent blood concentration (µg/mL) | HER2 + cells (%) | HER3 + cells (%) | Proportion of cells reached by agent (%) | Effect determination (score) |
|---|---|---|---|---|---|
| Specimen A | 570 | 0 | 0 | 0 | 0 |
| Specimen B | 650 | 30 | 5 | 25 | 1 |
| Specimen C | 580 | 60 | 0 | 50 | 2 |
| Specimen D | 500 | 100 | 5 | 80 | 3 |
| Specimen E | 470 | 0 | 80 | 0 | 0 |
| Specimen F | 570 | 30 | 70 | 10 | 0 |
| Specimen G | 620 | 60 | 70 | 25 | 1 |
| Specimen H | 540 | 100 | 80 | 40 | 2 |
| Specimen I | 320 | 0 | 5 | 0 | 0 |
| Specimen J | 310 | 30 | 5 | 15 | 0 |
| Specimen K | 210 | 60 | 0 | 20 | 1 |
| Specimen L | 300 | 100 | 0 | 50 | 2 |

### (Results and discussion)

From Fig. 1, it can be seen that the blood concentration of trastuzumab varies greatly for each specimen. This is considered to be due to the fact that a metabolic rate for an antibody medicine differs for each specimen.

From Fig. 2, it can be seen that an increase in the proportion of cells reached by trastuzumab corresponding to HER2 is observed in both the HER3 low expression group and high expression group, but the increase rate of the proportion of cells reached by trastuzumab corresponding to HER2 is suppressed in the HER3 high expression group.

From these results, it can be inferred that the higher the expression level of HER2, the easier the trastuzumab targeting HER2 is taken into the tissue, whereby the proportion of cells reached is increased, that is, the proportion of cells reached by drug becomes higher, and trastuzumab uptake in HER2 is suppressed by HER3. In addition, from Table 1, it was confirmed that, among specimens expressing the same level of HER2 and measuring the same level of trastuzumab blood concentration, the proportion of cells reached was low in cases where HER3 was high (E to H) as compared to cases where HER3 was low (A to D).

From the above, it can be inferred that the therapeutic effect can be assessed by, in addition to quantifying HER2 (target molecule) in a tumor tissue before antibody medicine administration, combining the expression level of a target-related molecule, the blood concentration, and the proportion of cells reached by the antibody medicine. Specifically, since the proportion of cells reached by the antibody medicine and the treatment result are correlated, the therapeutic effect can be predicted with high accuracy, based on the proportion of cells reached by the antibody medicine. In addition to the quantification of the target molecule, it is possible to more accurately predict the proportion of cells reached by the antibody medicine from the expression level of the target-related molecule and the blood concentration.

### [Experimental Example 4]

### <Assessment Preparation 2>

Assessment was performed in the same manner as in Assessment Preparation 1, except that anti-HER2 aptamer-A was administered in place of trastuzumab (trade name; Herceptin (registered trademark)).

Anti-HER2 aptamer-A (HER2 antigen-recognizing RNA aptamer, RNA 5-AGCCGCGAGGGGAGGGAUAGGGUAGGGCGCGGCU-3, obtained by requesting synthesis to Hokkaido System Science Co., Ltd.) was administered to PDX model mice at 3 mg/kg.

Blood was collected from the above mice in the same manner as in the step (I), and the anti-HER2 aptamer-A in the blood was detected by a hybridization method. Specifically, a detection (detect) probe solution having an NH2 residue (5'-biotin-CCCGCGCC-3, obtained by requesting synthesis to Hokkaido System Science Co., Ltd.) was added to a hybridization plate prepared by spotting the following solid-phase side (capture) probe (5'-NH2-GCGCUCCC-3, obtained by requesting synthesis to Hokkaido System Science Co., Ltd.) on a 96-well plate using a spotter. The mouse plasma was added thereto and allowed to react to detect the anti-HER2 aptamer-A in the blood.

Further, a tissue slide was prepared in the same manner as in the step (II), and immunostaining was performed for HER-2 and HER-3 in the same manner as in the immunostaining A.

A tissue slide was prepared in the same manner as in the step (III), and stained by the following procedure.

### (Pretreatment of specimen)

After the prepared FFPE tissue slide was deparaffinized, washing was performed by replacing with water. Thereafter, a protease enzyme treatment (30°C, 5 min) and a dehydration treatment were performed.

A particle solution in which the blue PID staining solution prepared in Preparation Example 5, the detection side (detect) probe and a denaturing solution (formamide/SSC solution or the like) were previously mixed was dropped on the tissue slide that had been subjected to the specimen pretreatment process to perform a hybridization reaction at 75°C for 5 minutes and at 37°C for 16 hours. After washing with stringency, the tissue slide was encapsulated with a DAPI solution, and observed in the next step.

(Observation, photographing, and image processing) were performed in the same manner as in Experimental Example 2.

### Industrial Applicability

From the above results, it can be said that the therapeutic effect of the antibody medicine can be assessed based on the expression level of the target molecule, the expression level of the target-related molecule, the blood concentration, and the proportion of cells reached by medicine X, and it is possible to select the medicine X and to construct a dosing regimen in the clinical field based on the assessment.

## Claims

1. A method for assessing a medicine that specifically recognizes a target molecule, comprising step (P) of obtaining information on the target molecule, and further comprising:
at least one step selected from the following steps (A), (B) and (C) (excluding, however, step (A) alone and step (B) alone), wherein
(A) is a step of obtaining information on a target-related molecule,
(B) is a step of measuring blood concentration of the medicine that specifically recognizes a target molecule in a subject, and
(C) is a step of calculating a proportion of cells reached by the medicine that specifically recognizes a target molecule.

2. The method for assessing a medicine according to claim 1, wherein the information on a target-related molecule includes at least one of information on an expression level of the target-related molecule and information on localization of the target-related molecule.

3. The method for assessing a medicine according to claim 1 or 2, wherein the information on a target molecule includes at least one of information on an expression level of the target molecule and information on localization of the target molecule.

4. The method for assessing an antibody medicine according to any one of claims 1 to 3, wherein the target molecule is HER2, CTLA4, EGFR, PD-1, PDGFRA, PD-L1, VEGF, or VEGFR.

5. The method for assessing a medicine according to any one of claims 1 to 4, wherein the medicine that specifically recognizes a target molecule is an antibody medicine.

6. The method for assessing a medicine according to any one of claims 1 to 5, wherein the step (C) is a step of quantifying blood concentration of the antibody medicine using ELISA.

7. The method for assessing a medicine according to any one of claims 1 to 4, wherein the medicine that specifically recognizes a target molecule is a nucleic acid medicine.

8. The method for assessing a medicine according to claim 7, wherein the target molecule is PDGF-B, C5, MCP-1, SDF-1, Hepcidin, RTP801, Nucleocapsid, transthyretin, or HSP47.
